# EUROPEAN PATENT APPLICATION

(11) **EP 1 371 718 A1**
(43) Date of publication of application: **17.12.2003**
(21) Application number: 03253684.9
(22) Date of filing: 11.06.2003
(51) Int. Cl.: C11D 3/37

(54) **Polymeric nanoparticle formulations and their use as fabric care additives**

(30) Priority: 14.06.2002 US 389043 P; 30.09.2002 US 414595 P
(71) Applicant: ROHM AND HAAS COMPANY, Philadelphia, Pennsylvania 19106-2399 (US)
(72) Inventor: Ghosh, Tirthankar, Oreland, Pennsylvania 19462 (US); Keenan, Andrea Claudette, Plymouth Meeting, Pennsylvania 19462 (US)
(74) Representative: Kent, Venetia Katherine

(57) **Abstract**

A polymeric nanoparticle having a mean particle diameter of 1 to 10 nm which is useful as a fabric care additive in laundry detergent formulations to improve properties such as softening, crease-resistance, soil and stain removal, soil release, dye transfer, dye fixation, static control and anti-foaming is disclosed. These nanoparticles may be utilized with silicone compounds in the detergent formulation to enhance their fabric care properties or the nanoparticles may be functionalized with silicone groups to significantly enhance various fabric care properties of the detergent formulation.

## Description

This invention relates to compositions for treating fabrics. It specifically relates to the use of such compositions for treating fabrics to protect and otherwise impart various desirable properties to fabrics that are subjected to detergent washing, rinsing and drying in mechanical fabric washing operations.

The term "fabric care" means the protection of fabrics during machine washing operations against physical and chemical degradation as well as imparting beneficial properties to the fabrics, such as softening and crease-resistance.

The mechanical machine washing of fabrics leads to the physical and chemical degradation of the fabric's fibres. The alkalinity of detergents as well as certain enzymes used with detergents contributes to the chemical degradation of the fibres of the fabric. Mechanical action takes place during the various cycles in the washing operation, such as agitating, rinsing, spin-drying and tumble-drying. It is generally the combination of chemical and physical forces which contributes to fibre degradation. Fibre degradation leads to the formation of fibrils at the surface of the fabric, which causes the material to lose its radiance. Further, this contributes to decreasing strength of the fibres, which might eventually lead to the tearing of the fabric. Traditional approaches toward treating fabrics have involved adding compounds such as silicones, such as aminomodified silicone polyether compolymers, dimethicone copolyol methyl ethers, and dimethicone bisamino hydroxypropyl copolyols.

The cleaning of fabrics in a washing machine, which systematically includes one or more spin drying operations, significantly contributes to the creasing of the fabric materials. Creasing is exacerbated by the high compressive pressures exerted on the fabric during the energy intensive spin and tumble dry cycles that are part of the wash and rinse process of almost every commercial and domestic washing machine in use today.

Creasing results from the hydrogen bonding that forms between the fibres as they are forced against each other under the extreme pressures exerted during the spin cycles coupled with the generally alkaline aqueous environment of the washing machine. Some silicone based products, such as polydimethylsiloxanes, have been utilized, with some degree of success, in the past in an attempt to improve crease resistance. However, they are expensive and, if applied during the wash or rinse cycles, they tend to wash away from the fabric leaving little or no protection against creasing upon completion of the wash cycle.

A fabric care additive is disclosed in International Patent Application PCT/FR01/02649, published as US 2002/0065208 A1. This publication describes the use of very small nanoparticles or "nanolatex" particles having diameters of from 10 to 500 nm and preferably from 20 to 50 nm. These particles may be added at various phases of the washing or drying cycle.

It is believed that these nanoparticles are as prone to washing away as the previously mentioned polydimethylsiloxanes during the wash and rinse cycles. What is needed is a compound which will remain affixed to the fibres of the fabric in order to be able to impart the desired effect. The polymeric nanoparticles of the present invention satisfy this need.
The present invention provides a method for treating fabrics in a mechanical fabric washing operation comprising adding to the aqueous wash water a compound comprising a crosslinked polymeric nanoparticle (hereinafter, "PNPs") having a mean particle diameter of 1 to 10 nanometers, preferably from 2 to 8 nm, more preferably from 3 to 6 nm, the nanoparticles comprising as polymerized units at least one multi-ethylenically-unsaturated monomer. In a second aspect of the invention there is provided a method for improving the efficacy of silicone fabric care compounds in a mechanical fabric washing operation comprising adding to the aqueous wash water the PNPs of the present invention. In a third aspect of the invention there is provided a fabric care treatment comprising a method of adding the PNPs of the present invention which contain pendant silicone functional groups to the wash water of a mechanical fabric washing operation.

Within the context of the present invention, "fabric care" encompasses not only softening and crease resistance, but also the properties and functions of soil and stain removal, soil release, dye transfer, dye fixation, static control and anti-foam. The PNPs of the present invention may be incorporated into a detergent formulation and added to the wash water at the beginning of the wash cycle. Alternatively, they may be added separately during the rinse cycle, as is customary with traditional softening treatments. Also, the PNPs may be added prior to the start of the drying cycle, as required by the results desired and the particular formulation prepared.

The aqueous composition of the present invention includes an aqueous dispersion of polymeric particles having a mean diameter in the range of from 1 to 50 nanometers (nm), the particles including, as polymerized units, at least one multiethylenically unsaturated monomer and at least one ethylenically unsaturated water soluble monomer. As used herein, the term "dispersion" refers to a physical state of matter that includes at least two distinct phases wherein a first phase is distributed in a second phase, the second phase being a continuous medium. By "aqueous" herein is meant a medium that is from 50 to 100 weight % water, based on the weight of the aqueous medium.

The polymeric particles, referred to herein as polymeric nanoparticles ("PNPs"), are addition polymers, which contain, as polymerized units, at least one multiethylenically unsaturated monomer and at least one ethylenically unsaturated water soluble monomer.
Suitable multiethylenically unsaturated monomers useful in the present invention include di-, tri-, tetra-, or higher multifunctional ethylenically unsaturated monomers, such as, for example, divinyl benzene, trivinylbenzene, divinyltoluene, divinylpyridine, divinylnaphthalene divinylxylene, ethyleneglycol di(meth)acrylate, trimethylolpropane tri(meth)acrylate, diethyleneglycol divinyl ether, trivinylcyclohexane, allyl (meth)acrylate, diethyleneglycol di(meth)acrylate, propyleneglycol di(meth)acrylate, 2,2-dimethylpropane- 1,3-di(meth)acrylate, 1,3-butylene glycol di(meth)acrylate, 1,4-butanediol di(meth)acrylate, 1,6-hexanediol di(meth)acrylate, tripropylene glycol di(meth)acrylate, triethylene glycol di(meth)acrylate, tetraethylene glycol di(meth)acrylate, polyethylene glycol di(meth)acrylates, such as polyethylene glycol 200 di(meth)acrylate and polyethylene glycol 600 di(meth)acrylate, ethoxylated bisphenol A di(meth)acrylate, poly(butanediol) di(meth)acrylate, pentaerythritol tri(meth)acrylate, trimethylolpropane triethoxy tri(meth)acrylate, glyceryl propoxy tri(meth)acrylate, pentaerythritol tetra(meth)acrylate, dipentaerythritol monohydroxypenta(meth)acrylate, divinyl silane, trivinyl silane, dimethyl divinyl silane, divinyl methyl silane, methyl trivinyl silane, diphenyl divinyl silane, divinyl phenyl silane, trivinyl phenyl silane, divinyl methyl phenyl silane, tetravinyl silane, dimethyl vinyl disiloxane, poly(methyl vinyl siloxane), poly(vinyl hydro siloxane), poly(phenyl vinyl siloxane), and mixtures thereof. The term "(meth)acrylic" includes both acrylic and methacrylic and the term "(meth)acrylate" includes both acrylate and methacrylate. Likewise, the term "(meth)acrylamide" refers to both acrylamide and methacrylamide. "Alkyl" includes straight chain, branched and cyclic alkyl groups.

Typically, the PNPs contain at least 1% by weight based on the weight of the PNPs, of at least one polymerized multiethylenically unsaturated monomer. Up to and including 99.5 weight % polymerized multiethylenically unsaturated monomer, based on the weight of the PNPs, is effectively used in the particles of the present invention. It is preferred that the amount of polymerized multiethylenically unsaturated monomer is from 1% to 80%, more preferably from 1% to 60%, most preferably from 1% to 25%, by weight based on the weight of the PNPs.

The PNPs further contain, as polymerized units, at least one water soluble monomer. By "water soluble monomer" herein is meant a monomer having a solubility in water of at least 7 weight %, preferably at least 9 weight %, and most preferably as least 12 weight %, at a temperature of 25 °C. Data for the water solubility of monomers is found, for example, in "Polymer Handbook" (Second Edition, J. Brandrup, E.H. Immergut, Editors, John Wiley & Sons, New York) and "Merck Index"(Eleventh Edition, Merck & Co, Inc., Rahway, New Jersey). Examples of water soluble monomers include ethylenically unsaturated ionic monomers and ethylenically unsaturated water soluble nonionic monomers. Typically, the amount of the polymerized water soluble monomer is at least 0.5 weight %, based on the weight of the PNPs. Up to and including 99 weight % polymerized water soluble monomer, based on the weight of the PNPs, can be effectively used in the particles of the present invention.

Ethylenically unsaturated ionic monomer, referred to herein as "ionic monomer" is a monomer that is capable of bearing an ionic charge in the aqueous medium in which the PNPs are dispersed. Suitable ionic monomers include, for example, acid-containing monomers, base-containing monomers, amphoteric monomers; quaternized nitrogen-containing monomers, and other monomers that can be subsequently formed into ionic monomers, such as monomers which can be neutralized by an acid-base reaction to form an ionic monomer. Suitable acid groups include carboxylic acid groups and strong acid groups, such as phosphorus containing acids and sulfur containing acids. Suitable base groups include amines. It is preferred that the amount of polymerized ionic monomer based on the weight of the PNPs is in the range from 0.5 to 99 weight %, more preferably in the range of from 1 to 50 weight %, even more preferably from 3 to 40 weight %, and most preferably from 5 to 25 weight %.

Suitable carboxylic acid-containing monomers include carboxylic acid monomers, such as (meth)acrylic acid, acryloxypropionic acid, and crotonic acid; dicarboxylic acid monomers, such as itaconic acid, maleic acid, fumaric acid, and citraconic acid; and monomers which are half esters of dicarboxylic acids, such as monomers containing one carboxylic acid functionality and one C₁₋₆ ester. Preferred are acrylic acid and methacrylic acid. Suitable strong acid monomers include sulfur acid monomers, such as 2-acrylamido-2-methyl propane sulfonic acid, styrene sulfonic acid, vinyl sulfonic acid, sulfoethyl (meth)acrylate, sulfopropyl (meth)acrylate, 2-acrylamido-2-methyl propane sulfinic acid, styrene sulfinic acid, and vinyl sulfinic acid; and phosphorus acid monomers, such as 2-phosphoethyl (meth)acrylate, vinyl phosphoric acid, and vinyl phosphinic acid. Other acid monomers include terminally unsaturated acid containing macromonomers as disclosed in U.S. Patent No. 5,710,227. Phosphorus acid monomers are desirable as they can provide improved adhesion to certain substrates (e.g., metal).

Suitable base-containing monomers include monomers having amine functionality, which includes N,N-dimethylaminoethyl (meth)acrylate, N,N-diethylaminoethyl (meth)acrylate, N-t-butylaminoethyl (meth)acrylate, N,N-dimethylaminopropyl (meth)acrylamide, p-aminostyrene, N,N-cyclohexylallylamine, allylamine, diallylamine, dimethylallylamine, N-ethyldimethylallylamine, crotyl amines, and N-ethylmethallylamine; monomers having pyridine functionality, which includes 2-vinylpyridine and 4-vinylpyridine; monomers having piperidine functionality, such as vinylpiperidines; and monomers having imidazole functionality, which includes vinyl imidazole. Other suitable base-containing monomers include oxazolidinylethyl (meth)acrylate, vinylbenzylamines, vinylphenylamines, substituted diallylamines, 2-morpholinoethyl (meth)acrylate, methacrylamidopropyl trimethyl ammonium chloride, diallyl dimethyl ammonium chloride, 2-trimethyl ammonium ethyl methacrylic chloride, and the like.

Suitable amphoteric monomers include N-vinylimidazolium sulfonate inner salts and N,N-Dimethyl-N-(3-methacrylamidopropyl)-N-(3-sulfopropyl) ammonium betaine.

Suitable functional monomers, in which the functionality is subsequently formed into an acid or base include monomers containing: an epoxide functionality, such as glycidyl (meth)acrylate and allyl glycidyl ether; an anhydride, such as maleic anhydride; an ester; and a halide. Suitable halide-containing functional monomers include vinylaromatic halides and halo-alkyl(meth)acrylates. Suitable vinylaromatic halides include vinylbenzyl chloride and vinylbenzyl bromide. Other suitable functional monomers include allyl chloride, allyl bromide, and (meth)acrylic acid chloride. Suitable halo-alkyl(meth)acrylates include chloromethyl (meth)acrylate.

Multiethylenically unsaturated monomers that are also water soluble monomers are alternatively used to prepare the PNPs. In such embodiments, these monomers are classified for the purposes of the present invention as both a multiethylenically unsaturated monomer and a water soluble monomer. An example of a water soluble, multiethylenically unsaturated monomer is the phosphodi(ethyl methacrylate).

Ethylenically unsaturated water soluble nonionic monomers are referred to herein as "water soluble nonionic monomers". Examples of water soluble nonionic monomers include hydroxyalkyl (meth)acrylates such as hydroxyethyl (meth)acrylate and hydroxypropyl (meth)acrylate; poly(alkylene oxide) esters of (meth)acrylic acid such as poly(ethylene oxide)₂₀ methacrylate and poly(propylene oxide)₁₅₀ acrylate; acrylamide; and methacrylamide. It is preferred that the amount of polymerized water soluble nonionic monomer based on the weight of the PNPs is in the range from 0.5 to 99 weight %, more preferably in the range of from 20 to 90 weight %, even more preferably from 30 to 80 weight %, and most preferably from 40 to 70 weight %.

The PNPs optionally contain, as polymerized units, one or more third monomers that are not multiethylenically unsaturated monomers and are not water soluble monomers. Suitable third monomers include C₁ -C₂₄ alkyl (meth)acrylates, such as methyl (meth)acrylate, ethyl (meth)acrylate, propyl (meth)acrylate, butyl (meth)acrylate, isobutyl (meth)acrylate, hexyl (meth)acrylate, cyclohexyl (meth)acrylate, 2-ethylhexyl (meth)acrylate, octyl (meth)acrylate, decyl (meth)acrylate, dodecyl (meth)acrylate, pentadecyl (meth)acrylate, hexadecyl (meth)acrylate, octadecyl (meth)acrylate, and nonadecyl (meth)acrylate, and mixtures thereof. Other suitable third monomers include 2-hydroxyethyl (meth)acrylate, 2-hydroxypropyl (meth)acrylate, 1-methyl-2-hydroxyethyl (meth)acrylate, 2-hydroxybutyl (meth)acrylate; vinyl acetate; vinyl versatate; diisobutylene; ureido containing monomers such as N-(ethyleneureidoethyl)-4-pentenamide, N-(ethylenethioureido-ethyl)-10-undecenamide, butyl ethyleneureido-ethyl fumarate, methyl ethyleneureido-ethyl fumarate, benzyl N-(ethyleneureido-ethyl) fumarate, and benzyl N-(ethyleneureido-ethyl) maleamate; vinylaromatic monomers, such as styrene, α-methylstyrene, vinyltoluene, p-methylstyrene, ethylvinylbenzene, vinylnaphthalene, vinylxylenes, and nonylphenoxy propenyl polyethoxylated alcohol. The vinylaromatic monomers also include their corresponding substituted counterparts, such as halogenated derivatives, i.e., containing one or more halogen groups, such as fluorine, chlorine or bromine; and nitro, cyano, (C₁ -C₁₀ )alkoxy, halo(C₁ -C₁₀ )alkyl, (C₁ -C₁₀ )alkoxy, carboxy, and the like.

The PNPs have a mean diameter in the range of from 1 to 50 nm, preferably in the range of from 1 to 40 nm, more preferably from 1 to 30 nm, even more preferably from 1 to 25 nm, even further preferably from 1 to 20 nm, and most preferably from 1 to 10 nm. It is further typical that the PNPs have a mean particle diameter of at least 1.5 nm, preferably at least 2 nm. One method of determining the particle sizes (mean particle diameter) of the PNPs is by using standard dynamic light scattering techniques, wherein the correlation functions are converted to hydrodynamic sizes using LaPlace inversion methods, such as CONTIN.

Typically, PNPs including as polymerized units, less than 10 weight % multiethylenically unsaturated monomer, have a glass transition temperature from -90 °C to 170 °C for the composition in the absence of the polymerized multiethylenically unsaturated monomer, as determined by a modulated DSC measurement. PNPs containing as polymerized units, at least 10 weight % multiethylenically unsaturated monomer are considered to have glass transition temperatures of at least 50°C.

The PNPs of the present invention typically have an "apparent weight average molecular weight" in the range of 5,000 to 1,000,000, preferably in the range of 10,000 to 500,000 and more preferably in the range of 15,000 to 100,000. As used herein, "apparent weight average molecular weight" reflects the size of the PNP particles using standard gel permeation chromatography methods, e.g., using THF solvent at 40 °C, 3 Plgel™ Columns (Polymer Labs, Amherst, MA), 100 Angstrom (10 nm), 103 Angstroms (100 nm), 104 Angstroms (1 micron), 30 cm long, 7.8 mm ID, 1 milliliter per minute, 100 microliter injection volume, calibrated to narrow polystyrene standards using Polymer Labs CALIBRE ™ software.

The PNPs are optionally characterized as having suitable hydrophilicities that allow the PNPs to be dispersed into an aqueous medium. One method to characterize the hydrophilicity of the PNPs is to calculate the Hansch parameter. The Hansch parameter is calculated using a group contribution method. The monomer units forming the polymer are assigned a hydrophobicity contribution and the relative hydrophobicity of the polymer is calculated based on the weight average of the monomers in the polymer. Hansch and Fujita, *J. Amer. Chem. Soc.,* 86, 1616-1626 (1964); H. Kubinyi, *Methods and Principles of Medicinal Chemistry,* Volume 1, R. Mannhold et al., Eds., VCH, Weinheim (1993); C. Hansch and A. Leo, *Substituent Constants for Correlation Analysis in Chemistry and Biology,* Wiley, New York (1979); and C. Hansch, P. Maloney, T. Fujita, and R. Muir, *Nature,* 194. 178-180 (1962).

Values of the hydrophobicity contributions for several monomers are listed in Table 1.

**Table 1**

| Monomer | Hydrophobicity Contribution |
|---|---|
| ethyl acrylate | 2.11 |
| butyl acrylate | 3.19 |
| 2-ethyl hexylacrylate | 5.22 |
| styrene | 4.29 |
| methyl methacrylate | 1.89 |
| ethyl methacrylate | 2.43 |
| butyl methacrylate | 3.51 |
| isobornyl methacrylate | 2.22 |
| butadiene | 4.0 |
| acrylic acid | -2.52 |
| methacrylic acid | -2.2 |
| maleic anhydride | -3.5 |

Preferred PNPs have a Hansch parameter in the range of from -2.5 to 4, alternatively from -1 to 3.
The PNPs optionally contain other functional groups, which are provided by the polymerization of monomers containing those groups or precursor groups thereof. Functional groups are optionally attached to the PNPs by reacting the ionic group of the PNP with a suitable compound. For example, PNPs containing carboxylic acid groups are modified to contain pendant hydrophilic groups by reacting carboxylic acid groups with a suitable alcohol, such as a capped polyalkylene oxide. Alternatively, functional groups are affixed to the PNPs through non-radical reactions resulting in the formation of ionic or covalent bonds between a modifying compound containing the groups and complementary reactable groups covalently bound to the PNP as taught in U.S. Patent No. 5,270,380.

The complementary reactable groups in the PNP and modifying compound provide ionic or covalent bonding. Complementary ionic bonding includes acid-base interaction and ion pair bonding of negatively and positively charged atoms. Covalent bonding by complementary reactable groups includes, for example:
(a) acetoacetate-aldehyde; (b) acetoacetate-amine; c) amine-aldehyde; (d) amine-anhydride; (e) amine-isocyanate; (f) amine-epoxy; (g) aldehyde-hydrazide;
(i) acid-epoxy; (j) acid-carbodiimide; (k) acid-chloro methyl ester; (j) acid-chloro methyl amine;
(m) acid-anhydride; (n) acid-aziridine; (o) epoxy-mercaptan; and
(p) isocyanate-alcohol. The first or second reactable group in each pair is present either in the PNP or, alternatively, in the modifying compound.

A suitable method to prepare the aqueous composition containing the PNPs dispersed in an aqueous medium includes the steps of preparing a nonaqueous PNP dispersion containing the PNPs dispersed in at least one solvent; and combining the nonaqueous PNP dispersion with an aqueous medium. By "nonaqueous" herein is meant a medium that contains from zero to less than 50 weight % water, based on the weight of the nonaqueous medium. Aqueous compositions containing PNPs that include, as polymerized units, ionic monomers, are optionally partially or completely neutralized prior to, during, or after combining with the aqueous medium.

A suitable polymerization process to prepare the nonaqueous PNP dispersion is free radical solution polymerization of at least one multiethylenically unsaturated monomer, at least one water soluble monomer, and optionally, at least one third monomer. By "solution polymerization" herein is meant free radical addition polymerization in a suitable solvent for the polymer. By "suitable solvent for the polymer" herein is meant that linear random (co)-polymers having substantially similar polymerized monomer units to the PNPs, are soluble in the solvent. Another method for selecting a suitable solvent or mixture of solvents is on the basis of using solubility parameter analysis. According to such methods, the suitability of the solvent is determined by substantially matching the solubility parameters of the PNP and of the solvent, such as the Van Krevelen parameters of delta d, delta p, delta h and delta v. See, for example, Van Krevelen et al., Properties of Polymers. Their Estimation and Correlation with Chemical Structure, Elsevier Scientific Publishing Co., 1976; Olabisi et al., Polymer-Polymer Miscibility, Academic Press, NY, 1979; Coleman et al., Specific Interactions and the Miscibility of Polymer Blends, Technomic, 1991; and A. F. M. Barton, CRC Handbook of Solubility Parameters and Other Cohesion Parameters, 2nd Ed., CRC Press, 1991. Delta d is a measure of dispersive interactions, delta p is a measure of polar interactions, delta h is a measure of hydrogen bonding interactions, and delta v is a measure of both dispersive and polar interactions. Such solubility parameters are alternatively calculated, such as by the group contribution method, or determined experimentally, as is known in the art. A preferred solvent has a delta v parameter within 5 (joule per cubic centimeter)1/2, preferably within 1 (joule per cubic centimeter)1/2 of the polymer delta v parameter. Suitable solvents for the polymerization include organic solvents, such as hydrocarbons; alkanes; halohydrocarbons; chlorinated, fluorinated, and brominated hydrocarbons; aromatic hydrocarbons; ethers; ketones; esters; alcohols; and mixtures thereof. Particularly suitable solvents, depending on the composition of the PNP, include dodecane, mesitylene, xylenes, diphenyl ether, gamma-butyrolactone, ethyl acetate, ethyl lactate, propyleneglycol monomethyl ether acetate, caprolactone, 2-heptanone, methylisobutyl ketone, acetone, methyl ethyl ketone, diisobutylketone, propyleneglycol monomethyl ether, and alkylalcohols, such as isopropanol, decanol, and t-butanol.

The nonaqueous PNP dispersion is prepared by first charging a solvent, or alternatively, a mixture of solvent and some portion of the monomers, to a reaction vessel. The monomer charge is typically composed of monomers, an initiator, and a chain transfer agent. Typically, initiation temperatures are in the range of from 55 °C to 125 °C, although lower or higher initiator temperatures are possible using suitable low temperature or high temperature initiators known in the art. After the heel charge has reached a temperature sufficient to initiate polymerization, the monomer charge or balance of the monomer charge is added to the reaction vessel. The monomer charge time period is typically in the range of from 15 minutes to 4 hours, although both shorter and longer time periods are envisioned. During the monomer charge, the reaction temperature is typically kept constant, although it is possible to vary the reaction temperature. After completing the monomer mixture addition, additional initiator in solvent can be charged to the reaction and/or the reaction mixture may be held for a time.

Control of PNP particle size and distribution is achieved by one or more of such methods as choice of solvent, choice of initiator, total solids level, initiator level, type and amount of multi-functional monomer, type and amount of ionic monomer, type and amount of chain transfer agent, and reaction conditions.

Initiators useful in the free radical polymerization of the present invention include, for example, one or more of: peroxyesters, alkylhydroperoxides, dialkylperoxides, azoinitiators, persulfates, redox initiators and the like. The amount of the free radical initiator used is typically from 0.05 to 10% by weight, based on the weight of total monomer. Chain transfer reagents are optionally used to control the extent of polymerization of the PNPs useful in the present invention. Suitable chain transfer agents include, for example: alkyl mercaptans, such as dodecyl mercaptan; aromatic hydrocarbons with activated hydrogens, such as toluene; and alkyl halides, such as bromotrichloroethane.

In one method of preparing the aqueous composition of the present invention, at least a portion of the polymerized ionic monomer units of the PNPs are neutralized with at least one neutralizing agent to form an at least partially neutralized nonaqueous PNP dispersion. The polymerized ionic monomer units of the PNPs can be neutralized in a variety of ways. When the polymerized ionic monomer units are acidic, the neutralizing agent is typically a base. Likewise, when the polymerized ionic monomer units are basic, the neutralizing agent is typically an acid. Suitable bases include inorganic and organic bases. Suitable inorganic bases include the full range of the hydroxide, carbonate, bicarbonate, and acetate bases of alkali or alkaline metals. Suitable organic bases include ammonia, primary/secondary/tertiary amines, diamines, and triamines. Preferred basic neutralizing agents include sodium hydroxide, and ammonium hydroxide. Suitable acids include carboxylic acids, such as acetic acid; dicarboxylic acids; (di)carboxylic/hydroxyl acids; aromatic acids, such as benzoic acid; and a variety of other acids, such as boric, carbonic, citric, iodic, nitrous, nitric, periodic, phosphoric, phosphorous, sulfuric, sulfurous, and hydrochloric acid. None of the foregoing categories of bases and acids, are deemed to be limiting.

The amount of neutralizing agent required to neutralize the nonaqueous PNP dispersion is typically determined on a molar basis of neutralizing agent to polymerized ionic monomer units of the PNPs. Without being bound to a particular theory, the amount of polymerized ionic monomer units (i.e., level of charge) needed to stabilize the PNPs (i.e., maintain particle size during conversion from non-aqueous to aqueous medium) will vary as PNP composition and properties are varied. It is believed that the PNP hydrophobicity, Tg, crosslinking level, and type of counter-ion from the neutralizing agent are important variables. For providing stable aqueous PNP dispersions (i.e., wherein flocculation of the PNPs is minimized), the polymerized ionic monomer units are preferably at least 20%, more preferably at least 50%, even more preferably at least 80%, and most preferably at least 90% neutralized.

Neutralizing the PNPs is alternatively carried out in a variety of ways. In one method, the nonaqueous PNP dispersion is added to a solution containing the neutralizing agent while stirring. Preferably, the neutralizing agent is added as an aqueous solution over time while stirring the nonaqueous PNP dispersion to provide an at least partially neutralized nonaqueous PNP dispersion.

In one method of preparing the aqueous composition containing dispersed PNPs, the at least partially neutralized nonaqueous PNP dispersion is combined with an aqueous medium. The aqueous medium optionally contains the neutralizing agent(s) for neutralizing the PNPs, in which case the nonaqueous PNP dispersion is capable of being simultaneously neutralized and combined with an aqueous medium. The aqueous medium optionally contains surfactants, which are capable of altering the stability of the PNPs, or of altering other properties of the resulting aqueous PNP dispersion, such as its surface tension.

The sequence of admixing the partially neutralized nonaqueous PNP dispersion and the aqueous medium is not critical. Various methods and equipment, which are suitable for mixing are described in *The Chemical Engineer's Handbook, 5th Edition,* Perry and Chilton, Eds., McGraw-Hill, Ch. 21, 1973. Typically, the aqueous medium is continuously stirred while adding the partially neutralized nonaqueous PNP dispersion to it in order to ensure that the solvent is intimately mixed with the aqueous medium, which minimizes flocculation of the PNPs.

Suitable weight percentages of the PNPs in the aqueous composition, based on total weight of the aqueous composition, are typically from 1 to 90 weight %, more typically from 2 to 75 weight %, even more typically from 4 to 65 weight %, further more typically from 8 to 55 weight %, and most typically from 10 to 45 weight %.

While the preparation of the aqueous composition of the present invention does not require the use of surfactants, and it is typical that the nonaqueous PNP dispersions are substantially free of surfactants, surfactants are optionally included. When present, the amount of surfactants is typically less than 3 weight percent, more typically less than 2 weight percent, even more typically less than 1 weight percent, further typically less than 0.5 weight percent, and even further typically less than 0.2 weight percent, based on total weight of the PNPs.

The aqueous composition is optionally treated to remove at least a portion of the solvent and optionally water, to increase the solids content of the PNPs. Suitable methods to concentrate the PNPs include distillation processes, such as forming azeotropes of water and a suitable solvent; evaporation of solvent or water; drying the aqueous composition by freeze drying or spray drying; solvent extraction techniques; and ultrafiltration techniques. Preferably at least 25 weight %, more preferably at least 50 weight %, even more preferably at least 75 weight %, and most preferably 100 weight % of the solvent is exchanged with water. Removal of the solvent is preferably carried out under conditions that minimize destabilization (i.e., flocculation) of the PNPs.

In an alternative method, the aqueous composition of this invention is prepared by a method including the steps of preparing a nonaqueous PNP dispersion containing the PNPs dispersed in at least one solvent that is both a suitable solvent for the PNPs and is compatible or miscible in water; and combining the nonaqueous PNP dispersion with an aqueous medium. Examples of such suitable solvents for acrylic-containing PNPs, which are also compatible or miscible with water, include isopropanol and ether alcohols (e.g., monobutyl ether of ethylene glycol and monoethyl ether of diethylene glycol). In this method, the PNPs do not require the addition of neutralizing agents to impart particle stability when combined with water.

Alternate embodiments of the aqueous compositions of the present invention have a wide range of PNP content. Typically, the PNP weight fractions range from 0.1 to 99 weight %, more typically from 1 to 90 weight %, even more typically from 2 to 75 weight %, further typically from 5 to 50 weight %, and most typically 10 to 40 weight %, based on the weight of the aqueous composition. Viscous, syrup-like, aqueous compositions typically result when the PNP weight fraction is greater than 25 weight %, while thin, liquid-like aqueous compositions are generally provided when the PNP weight fraction is less than 15 weight %. The correlation of viscosity and solid concentration is also affected by other parameters, such as composition and temperature.

The aqueous compositions of the present invention optionally contain other polymer particles that are not PNPs, such as emulsion polymer particles having a mean particle diameter of greater than 50 nm. The PNPs are present in the aqueous composition at a level of from 0.1 to 100 weight % PNPs based on the total weight of the polymer particles in the aqueous composition. The functional role of the PNPs determines the level of PNPs present. Some of the functional roles of the PNPs are, for example, as additives, as co-binders, as principal binders, and as sole binders. The amount of PNPs utilized ranges from a lower level for the former to a higher level of the latter.

In one embodiment, the PNPs are provided with other polymer particles, wherein the other polymer particles are prepared in the presence of the PNPs. In this embodiment, the PNPs are present in the aqueous composition at a level of from 1 to 99 weight %, preferably from 5 to 90 weight %, more preferably from 10 to 60 weight %, and most preferably from 20 to 60 weight %, based on the total weight of polymer in the aqueous composition.

The polymeric nanoparticles useful in the present invention may also be post-functionalized. Such post-functionalization may be by any techniques known in the art. Post-polymerization functionalization of the nanoparticles may be advantageous, such as in compatiblizing the particles with other ingredients in the detergent formulation.

The PNPs useful in the present invention may be functionalized with silicone groups by polymerizing acrylic monomers with silicones containing ethylene oxide/propylene oxide repeat units. PNPs containing silicon and/or poly(alkylene oxide) groups can be prepared by reacting PNPs containing reactive groups with silicone or poly(alkylene)oxide moieities that chemically interact with the PNP's reactive sites as described herein. The weight percentage of surface active monomers used for preparing the PNPs is in the range of from 1 to 90%, preferably from 2 to 75% and more preferably from 10 to 40%. Ideally, the reactive groups are on the surface or at least on the outer portion of the PNPs. Preferably, the PNPs have a Tg of less than 25C, more preferably less than 0C and most preferably less than -25C.

Silicone functionalized PNPs may be added into the wash cycle such that they will adhere to the fabric throughout the entire washing and drying operation. While not intending to be bound by theory, it is believed that the small size and dense cross-linked structure of the PNP give the particle its ability to remain with the fibres of the fabric throughout the entire washing and drying operation.

The following examples are presented to illustrate further various aspects of the present invention. EXAMPLE 1. Preparation of crosslinked polymeric nanoparticle.

A 500 mL reactor was fitted with a thermocouple, a temperature controller, a purge gas inlet, a water-cooled reflux condenser with purge gas outlet, a stirrer, and an addition funnel. To the addition funnel was charged 201.60 g of a monomer mixture consisting of 18.00 g methyl methacrylate (100% purity), 2.00 g diethyleneglycol dimethacrylate (100% purity), 1.60 g of a 75% solution of t-amyl peroxypivalate in mineral spirits (Luperox 554-M-75), and 180.00 g diisobutyl ketone ("DIBK"). The reactor, containing 180.00 g DIBK was then flushed with nitrogen for 30 minutes before applying heat to bring the contents of the reactor to 75° C. When the contents of the reactor reached 75° C, the monomer mixture in the addition funnel was uniformly charged to the reactor over 90 minutes. Thirty minutes after the end of the monomer mixture addition, the first of two chaser aliquots, spaced thirty minutes apart and consisting of 0.06 g of a 75% solution of t-amyl peroxypivalate in mineral spirits (Luperox 554-M-75) and 2.00 g DIBK, was added. At the end of the second chaser aliquot, the contents of the reactor were held 2½ hours at 80° C to complete the reaction. The resulting polymer was isolated by precipitation with heptane, collected by filtration and dried under vacuum to yield a white powder. This material was redissolved in propyleneglycol monomethylether acetate. The nanoparticles thus formed had a particle size distribution of from 0.8 to 5.0 nm with mean of 1.4 nm as determined by dynamic laser light scattering and a molecular weight of about 22,642 g/mol with a number average molecular weight of about 14,601 g/mol and Mw/Mn distribution of 1.6 as measured by GPC.

### EXAMPLE 2. Preparation of crosslinked polymeric nanoparticle - AAEM/ALMA copolymer by a semi-batch emulsion polymerization process.

A monomer emulsion was made from a mixture of 17 g deionized water, 8.85 g of 28 % w/w solids ammonium lauryl sulfate ("ALS"), 12.4 g acetoacetoxyethyl methacrylate ("AAEM"), and 1.78 g allyl methacrylate ("ALMA"). A reaction kettle was then prepared with 600 g deionized water, 15.0 g of 28 % w/w solids ALS, and 0.15 g ammonium persulfate ("APS") in 1 mL deionized water. The reaction kettle was heated to 90° C while being purged with nitrogen. One half of the monomer emulsion was added to the reaction kettle with stirring at 200 rpm. After 20 minutes, the remaining monomer emulsion was added. The kettle temperature was kept at 90° C for 30 minutes, cooled to 55° C, and then a solution of 0.02 g t-butyl hydroxy peroxide ("t-BHP") in 1 mL of deionized water and a solution of 0.010 g sodium sulfoxylate formaldehyde ("SSF") in 1 mL of deionized water were added respectively. The reaction was then cooled to ambient temperature and the emulsion was filtered through 400 and 100 mesh sieves respectively.

The sample was isolated from water by freeze-drying to produce a white friable, free flowing powder. The resulting white powder was washed with copious amounts of doubly distilled and deionized water to remove most of the surfactant.

### EXAMPLE 3. Preparation of crosslinked polymeric nanoparticle - AAEM/ALMA copolymer prepared by a batch emulsion polymerization process.

A monomer emulsion was made from a mixture of 17 g deionized water, 8.85 g of 28% w/w solids ALS, 12.4 g AAEM, and 1.78 g ALMA in a bottle. A reaction kettle was then prepared with 600 g deionized water, 15.0 g of 28% w/w solids ALS, and 0.15 g APS in 1 mL deionized water. The reaction kettle was heated to 90° C while being purged with nitrogen. The monomer emulsion was added all at once to the reaction kettle with stirring at 200 rpm. After 30 minutes, the temperature of the reaction flask was cooled to 75° C, and then a solution of 0.02 g t-BHP in 1 mL of deionized water was added. The reaction was cooled further to 55° C, and a solution of 0.010 g SSF in 2 mL of deionized water was added. The reaction was cooled to ambient temperature and the emulsion was filtered through 400 and 100 mesh sieves respectively.

### EXAMPLE 4. Preparation of crosslinked polymeric nanoparticle prepared by a gradual-addition polymerization process.

A monomer emulsion was made from a mixture of 100 g water, 1.60 g of 28% w/w solids ALS, 68 g ethyl acrylate ("EA"), 17 g methyl methacrylate ("MMA"), 12.5 g divinyl benzene ("DVB"), and 5 g methacrylic acid ("MAA"). A reaction kettle containing 445 g water, 22.2 g of 28% w/w solids ALS and 0.37 g APS was heated to 85° C under a nitrogen atmosphere. The monomer emulsion was fed to the kettle over 90 minutes. The reaction was held at 85° C for 30 minutes after the end of the feed, and then cooled to 65° C. After cooling, 1.33g of 10% iron sulfate (FeSO₄ ) was added. After 1 minute, 0.2 g of 70% t-BHP was added and after 2 minutes 0.10 g of 100% isoascorbic acid ("IAA") and the reaction held for 15 minutes. A second chaser system was added in the same sequence and over the same time period. The reaction was then cooled to ambient temperature and filtered through a 400 mesh sieve.

### EXAMPLE 5: Preparation of Silane Functional PNP

A monomer mixture of BA/MMA/MATS*/MAA/TMPTMA (450g: 39/31/10/10/10 w/w in 112g isopropanol) and Triganox® 125-C75 (9g) was added over 120 minutes to isopropanol (2325g) at 79 C under nitrogen. The batch was held at 79 C for 30 minutes before addition of Triganox 125-C75 (9g in 22g isopropanol). After addition of two further aliquots of Triganox 125-C75 (9g in 22g isopropanol) were added at 30 minute intervals, the batch was held for 2.5 hours, then allowed to cool to room temperature. The resulting material was determined to have an average particle size of 9.6nm by GPC.
*MATS: Methacryloxytrimethoxypropylsilane

### EXAMPLE 6: Preparation of a siloxane functional PNP

A 5 L. reactor was fitted with a thermocouple, a temperature controller, a purge gas inlet, a water-cooled reflux condenser with purge gas outlet, and a mechanical stirrer. A monomer mixture was made of 67.5 g butyl acrylate, 126.9 g methyl methacrylate, 27.0 g hydroxy ethyl methacrylate, 48.6 g methacrylic acid, 30 g trimethylolpropane trimethacrylate, 75 g CMS-222, 12 g of a 75% solution of t-amyl Peroxypivalate (Trigonox 125-C75), and 75 g isopropanol (IPA). The reactor, containing 1550 g IPA was heated to 79oC while purged with nitrogen for at least 30 minutes. When the contents of the reactor reached 79oC, the monomer mixture was pumped into the reactor over 120 minutes. Thirty minutes after the end of the monomer mixture feed, the first of three chaser aliquots, spaced thirty (30) minutes apart and consisting of 6 g of 75% solution of t-amyl Peroxypivalate (Trigonox 125-C75) and 15 g of IPA, was added. The contents of the reactor were held 2 hours at 79°C to complete the reaction. The PNP thus formed had an average particle size of 8.5 nm as determined by dynamic light scattering and a weight average molecular weight of 28,472 g/mol determined by GPC.
The CMS-222 (obtained from Gelest) is a carbinol containing methylsiloxane-dimethylsiloxane copolymer with a MW of 5500-6500.

### EXAMPLE 7: Crease Resistance Evaluation

Non-functionalized PNPs were combined with a commercial polymeric silicone to display the synergistic effect of these particles with silicone polymer detergent additives. The commercial silicone additive is a polyurethane-silicone hybrid containing 75% polysiloxane diol and is effective alone as an antiwrinkle aid for fabrics. However, it is very expensive and tends to rinse off the fabric very easily. It is known that silicone polymers impart lubricity to the fabric thereby reducing the occurrence of hydrogen bonding between fabric particles. This results in a reduction in fabric creasing. The following table displays the effects of adding increasing amounts of non-functionalized PNPs to the commercial product as shown by improved lubrication properties. Values are measured by the dynamic tensile loss modulus (E").
Cotton cloth, as would typically be used for clothing material, was cut into 14 x 15 inch segments. The cloth segments were run through a typical domestic washing machine cycle. Upon removal, each cloth segment was sprayed with approximately 5g of a solution containing the various percentages of active ingredients shown in the table below dissolved in ethanol. Due to the flammability of the ethanol, the cloth samples remained in a bundle at room temperature over night before being placed in a conventional domestic dryer.

**Table 7.1**

| **SAMPLE** | **PERCENT ACTIVE ON FABRIC** | **E" (dynes/cm2)** |
|---|---|---|
| Control | | 3.0 x 10⁷ |
| PNP* | 1.67 | 3.52 x 10⁷ |
| PNP | 0.167 | 3.50 x 10⁷ |
| PNP | 0.0167 | 3.40 x 10⁷ |
| PNP + Comm.Prod. | .83 + .83 | 3.42 x 10⁷ |
| PNP + Comm.Prod. | .083 + .083 | 3.72 x 10⁷ |
| PNP + Comm.Prod. | .0083 + .0083 | 4.02 x 10⁷ |

| | | |
|---|---|---|
| * PNP is a polymeric nanoparticle consisting of a 50:50 mix of MMA:TMPTMA mean particle diameter=8 nm | | |

The foregoing table shows the effectiveness of the PNP, even at low concentrations.

### EXAMPLE 8: Fabric Softness Evaluation

The purpose of this test is to evaluate the efficacy of polymeric nanoparticles as softeners in fabric washing and drying operations. Cotton terry cloth material is first cut into 13 x 15 inch segments for use as samples. The samples will then be washed and dried in conventional domestic automatic washers and dryers. The cloth samples are treated as shown in the table below. After drying, the cloth samples are evaluated on a scale of 1 to 5 for softness, with 1 feeling the harshest and 5 feeling the softest.

**TABLE 8.1**

| **SAMPLE** | **SOFTENER** | **RATING** |
|---|---|---|
| 1 | None | 1 |
| 2 | Control* | 5 |
| 3 | 1.6% actives PNP** | 5 |
| 4 | .16% actives PNP | 5 |
| 5 | .016% actives PNP | 4 |

| | | |
|---|---|---|
| * Control: Snuggle® commercial fabric softener | | |
| ** PNP: 50:50 mix of MMA:TMPTMA, mean particle diameter=8nm | | |

## Claims

1. A method for treating fabrics in a fabric washing and drying operation comprising adding polymeric nanoparticles, wherein the polymeric nanoparticles comprise at least one multi-ethylenically unsaturated monomer as polymerized units and having a mean particle diameter of 1 to 10 nanometers.

2. The method of claim 1 wherein the polymeric nanoparticles further comprise silicone functional groups.

3. The method of claim 1 wherein treating fabrics comprises one or more of the properties selected from the group consisting of softening, crease-resistance, soil removal, stain removal, soil release, dye transfer, dye fixation, static control and anti-foaming.

4. The method of claim 1 wherein the polymeric nanoparticles are added to the water at the beginning of the washing operation.

5. The method of claim 1 wherein the polymeric nanoparticles are added during the drying operation.

6. A process to enhance the efficacy of silicone fabric care compounds in a laundry detergent formulation comprising adding polymeric nanoparticles to the laundry detergent formulation, wherein the polymeric nanoparticles comprise at least one multi-ethylenically unsaturated monomer as polymerized units and have a mean particle diameter of 1 to 10 nanometers.
